# EUROPÄISCHE PATENTANMELDUNG

(11) **EP 2 055 693 A1**
(43) Veröffentlichungstag der Anmeldung: **06.05.2009**
(21) Anmeldenummer: 07119476.5
(22) Anmeldetag: 29.10.2007
(51) Int. Cl.: C07C 45/62, C07C 49/517, C07D 317/72

(54) **Verfahren zur Herstellung von substituierten und unsubstituierten Cyclohexanonmonoketalen**

(71) Anmelder: Bayer CropScience AG, 40789 Monheim (DE)
(72) Erfinder: Die Erfindernennung liegt noch nicht vor

(57) **Zusammenfassung**

Die vorliegende Erfindung betrifft ein Verfahren zur Herstellung und Isolierung bekannter substituierter und unsubstituierter 1,4-Cyclohexanonmonoketale der Formel (I).
Die Verbindungen (I) werden durch Hydrierung von Verbindungen der Formel (II) in Gegenwart eines geeigneten Metallkatalysators, gegebenenfalls eines Lösungsmittels und gegebenenfalls eines Additives hergestellt.

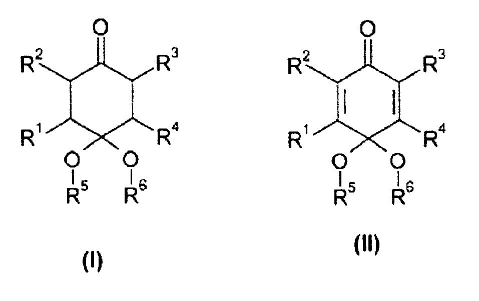

## Beschreibung

Die vorliegende Erfindung betrifft ein neues Verfahren zur Herstellung und Isolierung bekannter substituierter und unsubstituierter 1,4-Cyclohexanonmonoketale.

Substituierte und unsubstituierte Cyclohexanonmonoketale sind wichtige Ausgangsstoffe für die Synthese von Pflanzenschutz- und Pharmawirkstoffen wie beispielsweise dem Migränetherapeutikum Frovatriptan.

Zur Herstellung von Cyclohexanonmonoketalen sind in der Literatur eine Reihe verschiedener Verfahren bekannt:

Beispielsweise wird in US2004/0230063 die schwefelsäurekatalysierte Monoketalisierung von 1,4-Cyclohexandion mit einem Äquivalent Neopentylglykol beschrieben, wobei ein schwer trennbares Gemisch aus Dion, Monoketal und Bisketal entsteht. Daraus resultieren sehr aufwendige Aufarbeitungen, was für einen ökonomischen Prozess im technischen Maßstab sehr nachteilig ist. Auch die Verwendung anderer Diole ermöglicht keine selektive Monoketalisierung und Vereinfachung der Aufarbeitung.

In J. Org. Chem. 1983 48, 129-131 wird die Monoketalisierung von 1,4-Cyclohexandion mit 1,4-Butandiol beschrieben, wobei auch hier ein Gemisch aus Mono- und Bisketal erhalten wird. Darüber hinaus beträgt die isolierte Ausbeute nach einer aufwendigen Aufarbeitung lediglich 59 % d. Th. und zudem ist die Reaktion schwer im technischen Maßstab durchführbar. Weiterhin zu erwähnen ist, dass die Ausgangsverbindung 1,4-Cyclohexandion ein teurer Baustein ist.

Eine weitere Methode zur Herstellung von Monoketalen wird in einer Umsetzung von 1,4,9,12-Tetraoxadispiro[4.2.4.2]tetradecan mit 1,4-Cyclohexandion in Bull. Soc. Chim. Fr. 1983, 3-4, 87-88 beschrieben. Allerdings ist die Ausbeute für dieses Verfahren mit 74 % angegeben und erfordert eine sehr aufwendige und im technischen Maßstab teure Aufarbeitung.

Ferner werden in der Literatur kontrollierte Monodeketalisierungen von Bisketalen beschrieben. Beispielsweise wird in Synthesis 1987, 37-40 über die Monodeketalisierung ohne Lösungsmittel mittels Eisen(III)chlorid auf Kieselgel berichtet. Hierbei ist bemerkenswert, da das Bisketal 1,4,9,12-Tetraoxadispiro[4.2.4.2]tetradecan ein Feststoff ist. Auch dieses Verfahren besitzt somit die oben geschilderten Nachteile und die technische Realisierung eines solchen Verfahrens ist daher schwer praktikabel.

Im Hinblick auf die vorstehend geschilderten Nachteile und Probleme besteht dringend Bedarf für ein vereinfachtes, technisch und ökonomisch durchführbares Verfahren zur selektiven Herstellung von substituierten und unsubstituierten 1,4-Cyclohexanonmonoketalen im technischen Maßstab.

Es wurde gefunden, dass man Verbindungen der Formel (I) 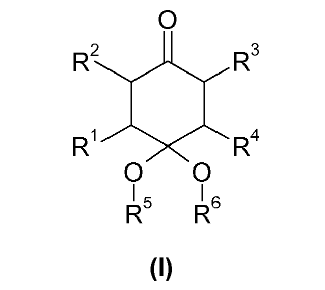 in welcher
R¹, R², R³, R⁴ unabhängig voneinander für Wasserstoff oder für jeweils gegebenenfalls einfach oder mehrfach substituiertes C₁-C₄-Alkyl oder Cyclopropyl stehen,
R⁵ und R⁶ unabhängig voneinander für C₁-C₈-Alkyl oder für Cycloalkyl stehen, oder
R⁵ und R⁶ gemeinsam für -CH₂-, -CH₂CH₂-, -CH₂CH₂CH₂-, -CH₂CH₂CH₂CH₂-, -CHCH₃CH₂CH CH₃CH₂-, -CH₂C(CH₃)₂CH₂, CH₂OCH₂-, -CH₂OCH₂CH₂- oder -CH₂CH₂OCH₂-stehen
durch Hydrierung von Verbindungen der Formel (II) 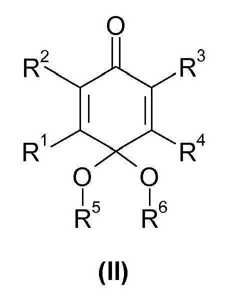 in welcher
R¹, R², R³, R⁴, R⁵, R⁶ die oben angegebenen Bedeutungen haben
in Gegenwart eines geeigneten Metallkatalysators, gegebenenfalls eines Lösungsmittels und gegebenenfalls eines Additives erhält.

In den allgemeinen Formeln (I) und (II) stehen die Substituenten

R¹, R², R³, R⁴ unabhängig voneinander bevorzugt für Wasserstoff oder für Methyl, Ethyl, i-Propyl, t-Butyl oder Cyclopropyl,

R⁵ und R⁶ unabhängig voneinander bevorzugt für Methyl, Ethyl, i-Propyl, t-Butyl oder für Cyclopropyl, Cyclobutyl, Cyclopentyl, oder Cyclohexyl oder

R⁵ und R⁶ gemeinsam bevorzugt für -CH₂-, -CH₂CH₂-, -CH₂CH₂CH₂-, -CH₂CH₂CH₂CH₂-, -CHCH₃CH₂CH CH₃CH₂- oder -CH₂C(CH₃)₂CH₂-.

In den allgemeinen Formeln (I) und (II) stehen die Substituenten

R¹, R², R³, R⁴ unabhängig voneinander besonders bevorzugt für Wasserstoff, Methyl oder Ethyl,

R⁵ und R⁶ unabhängig voneinander besonders bevorzugt für Methyl, Ethyl, i-Propyl oder t-Butyl oder

R⁵ und R⁶ gemeinsam besonders bevorzugt für -CH₂-, -CH₂CH₂-, -CH₂CH₂CH₂-, -CHCH₃CH₂CHCH₃CH₂- oder -CH₂C(CH₃)₂CH₂-.

In den allgemeinen Formeln (I) und (II) stehen die Substituenten

R¹, R², R³, R⁴ unabhängig voneinander ganz besonders bevorzugt für Wasserstoff oder Methyl, (hervorgehoben für Wasserstoff),

R⁵ und R⁶ unabhängig voneinander ganz besonders bevorzugt für Methyl, oder

R⁵ und R⁶ gemeinsam ganz besonders bevorzugt für -CH₂CH₂- oder -CH₂CH₂CH₂-.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden.

Insbesondere bevorzugt ist die Verbindung der Formel (I-1) 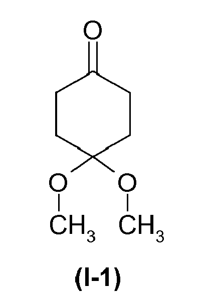 die durch Hydrierung der Verbindung der Formel (II-1) 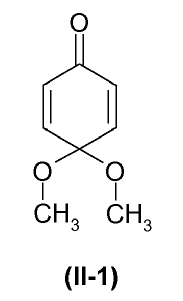 in Gegenwart eines geeigneten Metallkatalysators, eines gegebenenfalls Lösungsmittels und gegebenenfalls eines Additives erhalten wird.

Weiterhin insbesondere bevorzugt ist die Verbindung der Formel (1-2) 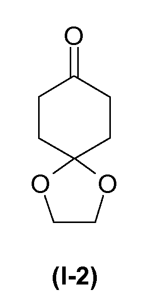 die durch Hydrierung der Verbindung der Formel (II-2) 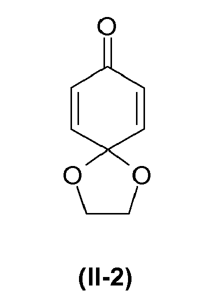 in Gegenwart eines geeigneten Metallkatalysators, gegebenenfalls eines Lösungsmittels und gegebenenfalls eines Additives erhalten wird.

Weiterhin insbesondere bevorzugt ist die Verbindung der Formel (I-3) 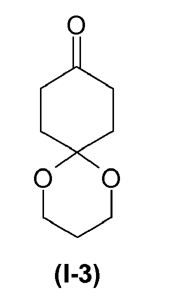 die durch Hydrierung der Verbindung der Formel (II-3) 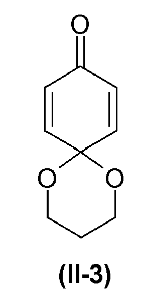 in Gegenwart eines geeigneten Metallkatalysators, gegebenenfalls eines Lösungsmittels und gegebenenfalls eines Additives erhalten wird.

Die Verbindungen der Formeln (I) und (II) sind literaturbekannt.

Man erhält die Verbindungen der Formel (II) durch Monodeketalisierung von Bisketalen nach bekannten Methoden der Ketalhydrolyse in Gegenwart einer katalytischen Menge einer organischen oder anorganischen Säure oder in einem Lösungsmittelgemisch (Protective Groups in Organic Synthesis, T. Greene and P. Wuts, Wiley-Interscience).

Die Hydrierung von Verbindungen der Formel (II) ist bisher in der Literatur auf sterisch sehr anspruchsvolle, stabile und vergleichsweise sehr teure cyclische Ketale beschränkt. Ein derartiges Verfahren beschreibt March et. al. in Tetrahedron Asymmetry 2003, 14, 2021-2032. Dabei wird über die palladiumkatalysierte Hydrierung von 2,3-Diphenylspiro[4.5]deca-6,9-dien-8-on in Toluol 2,3-Diphenylspiro[4.5]decan-8-on erhalten.

Bei der Hydrierung von Verbindungen der Formel (II) mit sehr teuren cyclischen Ketalen können nach Stand der Technik Nebenkomponenten, beispielsweise (A) und (B) in Schema 1, entstehen.

Es wurde nun überraschenderweise gefunden, dass auch sehr preiswerte und leicht verfügbare und somit industriell interessante Verbindungen der Formel (II) wie z. B. 4,4-Dimethoxycyclohexa-2,5-dien-1-on der Formel (II-1) oder 1,4-Dioxaspiro[4.5]deca-6,9-dien-8-on (II-2) oder 1,5-Dioxaspiro[5.5]undeca-7,10-dien-9-on (II-3) unter sehr milden Reaktionsbedingungen in Substanz oder in einem Lösungsmittel hydriert werden können.

Überraschenderweise wurde ebenfalls gefunden, dass sich die Bildung der Nebenkomponente der Formel (B) gemäß dem Schema 1 unter Zusatz einer Base und in geeigneten Lösungsmitteln wie beispielsweise Toluol, Methyl-Tetrahydrofuran oder Essigsäureethylester vermeiden bzw. auf Spuren reduzieren lässt. Mögliche Nebenkomponenten der Formel (B) können nach der Hydrierung bei der anschließenden Aufarbeitung mit Natriumhydroxid-Lösung entfernt werden.

Die Bildung von Nebenkomponente (A) wird bei hohen Drücken, in polaren Lösungsmitteln und längeren Reaktionszeiten bevorzugt und kann damit durch Einstellung der Reaktionsbedingungen reduziert oder sogar vollständig vermieden werden.

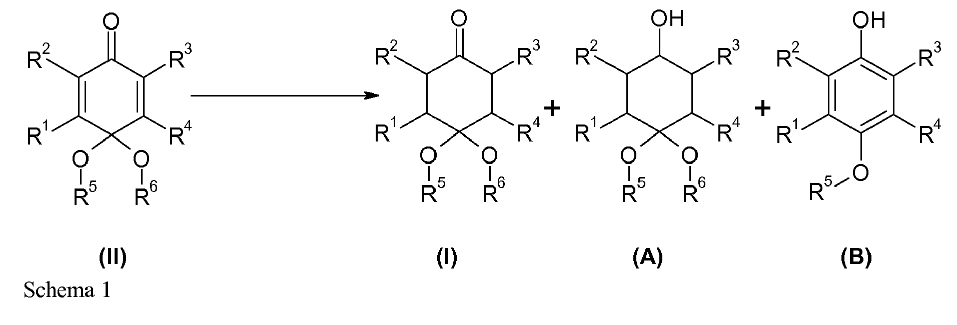

Somit ist es nach dem erfindungsgemäßen Verfahren möglich, die Bildung der Nebenkomponenten zu vermeiden bzw. stark zu reduzieren. Die Verbindungen der Formel (I) lassen sich daher in sehr guten Ausbeuten und Selektivitäten herstellen.

Die Hydrierung der Verbindungen der Formel (II) erfolgt bei Normaldruck oder Überdruck mit Wasserstoff in Gegenwart eines aktiven Metallkatalysators, eines unpolaren Lösungsmittels und eines Additives wie beispielsweise einer Base.

Als katalytisch aktive Metallverbindungen können alle dem Fachmann für diesen Zweck geläufige Katalysatoren in Betracht kommen. Vorzugsweise sind diese Verbindungen der Metalle der 8 bis 10. Nebengruppe des Periodensystems. Bevorzugt sind Metallkatalysatoren aus Palladium. Als Palladiumkatalysatoren bzw. -präkatalysatoren können beliebige Palladium(II)-verbindungen, Palladium(0)-verbindungen und Palladium auf einem beliebigen üblichen anorganischen Trägermaterial, wie beispielsweise Aluminiumoxid, Siliciumdioxid, Zirkondioxid, Titandioxid oder Kohlenstoff, besonders bevorzugt Palladium auf Aktivkohle, eingesetzt werden. Für das vorliegende Verfahren hat sich gezeigt, dass eine Menge von 0.0001 bis 5 mol% der katalytisch aktiven Metallverbindung (berechnet auf das Metall), bevorzugt 0.001 bis 3 mol% bezogen auf das Edukt ausreichen.

Als geeignete Basen können alle dem Fachmann für diesen Zweck in Frage kommenden anorganischen und organischen Basen wie beispielsweise Alkaliacetate, Alkali- und Erdalkalicarbonate, oder -hydrogencarbonate, Borax oder organische Basen wie Trialkylamine, beispielsweise 1,5-Diazabicyclo[5.4.0]undec-7-en, Triethylamin, Tri-n-butylamin oder Diisopropylethylamin oder ein Gemisch derselben verwendet werden. Bevorzugt ist der Einsatz von Triethylamin, Tri-n-butylamin oder Diisopropylethylamin. Für das vorliegende Verfahren kann die Stöchiometrie der eingesetzten Base in weiten Bereichen variieren und unterliegt im Allgemeinen keiner besonderen Beschränkung. So kann das molare Verhältnis der Base zu dem Edukt beispielsweise 0.001 bis 5, insbesondere 0.01 bis 2, speziell 0.01 bis 0.1 betragen. Der Einsatz größerer Mengen Base ist grundsätzlich möglich, bietet jedoch keine Vorteile.

Als Lösungsmittel können Wasser sowie alle dem Fachmann geläufigen organischen Verbindungen eingesetzt werden. Beispiele dafür sind Dioxan, Tetrahydrofuran, Methyl-Tetrahydrofuran, Ethylenglykoldimethylether, 1,2-Dimethoxyethan, Essigsäureethylester, Aceton, tert.-Butylmethylketon, Xylol, Toluol, Alkohole, wie z.B. Methanol oder Gemische davon. Die Lösungsmittel können in reiner Form oder produktenthaltende bzw. mit Produkt gesättigt eingesetzt werden. Bevorzugt sind Toluol, Essigsäureethylester und Methyl-Tetrahydrofuran als Lösungsmittel.

Die Hydrierung wird vorzugsweise bei einer Temperatur von 0-150 °C, besonders bevorzugt bei 20 bis 100 °C durchgeführt, wobei der Wasserstoffdruck üblicherweise von 1 bis 150 bar, vorzugsweise von 5 bis 100 bar beträgt. Eine Reaktionszeit von 0.01 bis 100 Stunden ist gewöhnlich ausreichend.

Die nachfolgenden Ausführungsbeispiele erläutern die Erfindung. Die Erfindung ist nicht auf die Beispiele beschränkt.

### Herstellungsbeispiele:

### Synthese von 3,3,6,6-Tetramethoxycyclohexa-1,4-dion [15791-03-4]

104 g (0.753 mol) 1,4-Dimethoxybenzol und 8 g (0.143 mol) Kaliumhydroxid werden in 500 ml Methanol gelöst. Anschließend wird in die Reaktionslösung eine platinierte Titananode und eine Nickelkathode eingetaucht. Die Reaktionsmischung wird bei Raumtemperatur in einer nicht getrennten Planschliffzelle bei 0.65 A und einer Zellspannung von 22 V mittels eines Laborpotentiostats TG 96 elektrolysiert. Die Umsetzung wird gaschromatographisch verfolgt. Nach Beendigung der Reaktion wird das Lösungsmittel unter vermindertem Druck weitgehend entfernt, der Rückstand in tert.-Butylmethylether aufgenommen und mit Wasser gewaschen. Man erhält 144 g (0.698 mol, 97 % Reinheit, 92.8 % Ausbeute) 3,3,6,6-Tetramethoxycyclohexa-1,4-dion.

### Synthese von 4,4-Dimethoxycyclohexa-2,5-dien-1-on (II-1) [935-50-2]

120 g (0.6 mol) 3,3,6,6-Tetramethoxycyclohexa-1,4-dion werden in einer Mischung von 480 ml Tetrahydrofuran, 60 ml Wasser und 6 ml Essigsäure 6 Stunden bei 70 °C gerührt. Nach vollständiger Monohydrolyse wird das Tetrahydrofuran im Vakuum abgezogen. Der wässrige Rückstand wird mit 100 ml NaHCO₃-Lösung versetzt und 2x mit 300 ml tert.-Butylmethylether extrahiert. Die vereinigten organischen Extrakte werden mit Na₂SO₄ getrocknet, über basischem Aluminiumoxid filtriert und einrotiert. Man erhält 88.4 g (0.562 mol, 98 % Reinheit, 95.7 % Ausbeute) 4,4-Dimethoxycyclohexa-2,5-dien-1-on (II-1).

### Synthese von 4,4-Dimethoxycyclohexanon (I-1) [56180-50-8]

154 g (0.958 mol, 95.7 % Reinheit) 4,4-Dimethoxycyclohexa-2,5-dien-1-on (II-1) und 10.8 g (0.083 mol) N,N-Diisopropylethylamin werden in 800 ml Methyl-Tetrahydrofuran gelöst und über 1.54 g Palladium 5 % auf Aktivkohle mit 100 bar Wasserstoff bis zur Druckkonstanz hydriert. Der Autoklav wird gekühlt, so dass die Reaktionstemperatur nicht über 30 °C steigt. Die Reaktionsmischung wird über Kieselgur abfiltriert. Nach Entfernung des Lösungsmittels erhält man 153 g (91.9 % Reinheit, 92 % Ausbeute) 4,4-Dimethoxycyclohexanon (I-1).

### Synthese von 1,4,9,12-Tetraoxadispiro[4.2.4.2]tetradeca-6,13-dien [35375-33-6]

1000 g (5 mol) 3,3,6,6-Tetramethoxycyclohexa-1,4-dion werden in 2000 ml 1,2-Ethandiol suspendiert. Bei 5 °C werden 0.6 g p-Toluolsulfonsäure zugegeben und die Suspension wird bei 5 °C noch 2 Stunden nachgerührt. Die Umsetzung wird gaschromatographisch verfolgt. Zur Vervollständigung der Fällung wird die Reaktionsmischung auf 0 °C abgekühlt. Der Feststoff wird abfiltriert, mit 1000 ml kaltem Wasser gewaschen und im Vakuum bei Raumtemperatur getrocknet. Man erhält 876 g (4.46 mol, 100 % Reinheit, 89 % Ausbeute) 1,4,9,12-Tetraoxadispiro[4.2.4.2]tetradeca-6,13-dien.

### Synthese von 1,4-Dioxaspiro[4.5]deca-6,9-dien-8-on (II-2) [35357-34-7]

813 g (4.12 mol) 1,4,9,12-Tetraoxadispiro[4.2.4.2]tetradeca-6,13-dien werden in einer Mischung von 1600 ml Tetrahydrofuran, 1600 ml Wasser und 32 ml Essigsäure 6 Stunden bei 64 °C gerührt. Die Umsetzung wird gaschromatographisch verfolgt. Nach vollständiger Monohydrolyse wird das Tetrahydrofuran im Vakuum abgezogen. Der wässrige Rückstand wird 2x mit 1000 ml Toluol extrahiert. Die vereinigten organischen Extrakte werden mit 20 g K₂CO₃ verrührt, mit Na₂SO₄ getrocknet und einrotiert. Man erhält 570 g (3.74 mol, 91 % Ausbeute) 1,4-Dioxaspiro[4.5]deca-6,9-dien-8-on (II-2).

### Synthese von 1,4-Dioxaspiro[4.5]decan-8-on (1-2) [4746-97-8]

10 g (0.66 mol) 1,4-Dioxaspiro[4.5]deca-6,9-dien-8-on und 0.7 g (5.4 mmol) N,N-Diisopropylethylamin werden in 200 ml Methyl-Tetrahydrofuran gelöst und über 0.1 g Palladium 5 % auf Aktivkohle mit 100 bar Wasserstoff bis zur Druckkonstanz hydriert. Der Autoklav wird gekühlt, so dass die Reaktionstemperatur nicht über 30 °C steigt. Die Reaktionsmischung wird über Kieselgur abfiltriert. Nach Entfernung des Lösungsmittels erhält man 9.4 g (91 % Ausbeute) 1,4-dioxaspiro[4.5]decan-8-on (1-2).

### Synthese von 1,5,10,14-Tetraoxadispiro[5.2.5.2]hexadeca-7,15-dien [77746-35-1]

50 g (0.25 mol) 3,3,6,6-Tetramethoxycyclohexa-1,4-dion werden in 500 ml 1,3-Propandiol suspendiert. Bei 0 °C werden 50 mg p-Toluolsulfonsäure zugegeben und die Suspension wird bei 0 °C noch 3 Stunden nachgerührt. Zur Vervollständigung der Fällung wird 300 ml gesättigter NaHCO₃-Lösung zugegeben. Der Feststoff wird abfiltriert, mit 200 ml kaltem Wasser gewaschen und im Vakuum bei Raumtemperatur getrocknet. Man erhält 46.1 g (0.207 mol, 82.3 % Ausbeute) 1,5,10,14-Tetraoxa-dispiro[5.2.5.2]hexadeca-7,15-dien.

### Synthese von 1,5-Dioxaspiro[5.5]undeca-7,10-dien-9-on (II-3) [67856-46-6]

95.2 g (0.425 mol) 1,5,10,14-Tetraoxadispiro[5.2.5.2]hexadeca-7,15-dien werden in einer Mischung von 340 ml Tetrahydrofuran, 170 ml Wasser und 5.1 ml Essigsäure 7 Stunden bei 70 °C gerührt. Nach vollständiger Monohydrolyse wird das Tetrahydrofuran im Vakuum abgezogen. Der wässrige Rückstand wird mit 100 ml NaHCO₃-Lösung versetzt und 2x mit 300 ml tert.-Butylmethylether extrahiert. Die vereinigten organischen Extrakte werden mit Na₂SO₄ getrocknet, über basischem Aluminiumoxid filtriert und einrotiert. Man erhält 58.0 g (0.35 mol, 83.8 % Ausbeute) 1,5-Dioxaspiro[5.5]undeca-7,10-dien-9-on.

### Synthese von 1,5-Dioxaspiro[5.5]undecan-9-on (1-3) [76626-13-6]

58 g (0.35 mol) 1,5-Dioxaspiro[5.5]undeca-7,10-dien-9-on (II-3) und 5.8 ml Triethylamin werden in 2 1 Toluol gelöst und über 5.8 g Palladium 10 % auf Aktivkohle mit 100 bar Wasserstoff bis zur Druckkonstanz hydriert. Der Autoklav wird gekühlt, so dass die Reaktionstemperatur nicht über 20 °C steigt. Die Reaktionsmischung wird über Kieselgur abfiltriert und nach Entfernung des Lösungsmittels erhält man 51.6 g (0.3 mol, 86.7 % Ausbeute) 1,5-Dioxaspiro[5.5]undecan-9-on.

## Patentansprüche

1. Verfahren zur Herstellung von Verbindungen der Formel (I) 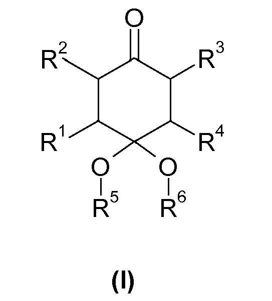 in welcher
R¹, R², R³, R⁴ unabhängig voneinander für Wasserstoff oder für jeweils gegebenenfalls einfach oder mehrfach substituiertes C₁-C₄-Alkyl oder Cyclopropyl stehen,
R⁵ und R⁶ unabhängig voneinander für C₁-C₈-Alkyl oder für Cycloalkyl stehen, oder
R⁵ und R⁶ gemeinsam für -CH₂-, -CH₂CH₂-, -CH₂CH₂CH₂-, -CH₂CH₂CH₂CH₂-, - CHCH₃CH₂CHCH₃CH₂-, -CH₂C(CH₃)₂CH₂-, -CH₂OCH₂-, -CH₂OCH₂CH₂-, - CH₂CH₂OCH₂- stehen
**dadurch gekennzeichnet, dass** man
Verbindungen der Formel (II) 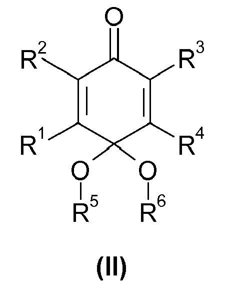 in welcher
R¹, R², R³, R⁴, R⁵, R⁶ die oben angegebenen Bedeutungen haben
in Gegenwart eines geeigneten Metallkatalysators, gegebenenfalls eines Lösungsmittels und gegebenenfalls eines Additives hydriert.

2. Verfahren zur Herstellung von Verbindungen der Formel (I) gemäß Anspruch 1, **dadurch gekennzeichnet, dass** eine organische Base als Additiv eingesetzt wird.
